# EUROPÄISCHE PATENTSCHRIFT

(11) **EP 1 816 117 B1**
(45) Veröffentlichungstag und Bekanntmachung des Hinweises auf die Patenterteilung: **05.11.2008**
(21) Anmeldenummer: 07001111.9
(22) Anmeldetag: 19.01.2007
(51) Int. Cl.: C07C 201/16, C07C 205/06

(54) **Verfahren zur Herstellung von Nitrobenzol**
Method for manufacturing nitrobenzole
Procédé destiné à la fabrication de nitrobenzène

(30) Priorität: 03.02.2006 DE 102006004943
(43) Veröffentlichungstag der Anmeldung: 08.08.2007
(73) Patentinhaber: Bayer MaterialScience AG, 51368 Leverkusen (DE)
(72) Erfinder: Knauf, Thomas, Dr., 41542 Dormage (DE); Gehlen, Franz-Ulrich, 47839 Krefeld (DE); Schmiedler, Jörg, Apt. 2901 200030 Shangai (CN); Pilarczyk, Klaus, 47809 Krefeld (DE); Drinda, Peter, 47829 Krefeld (DE)

(56) Entgegenhaltungen:
- EP-A2- 1 132 347
- DE-A1- 2 808 225
- US-A- 3 350 466

## Beschreibung

Die Erfindung betrifft ein kontinuierliches Verfahren zur Aufarbeitung von Rohnitrobenzol, bei dem man das rohe Nitrobenzol nach einander in einer sauren, in einer alkalischen und in einer neutralen Wäsche wäscht, wobei man bei der neutralen Wäsche die Nitrobenzol enthaltende organische Phase zunächst dispergiert und anschließend in einer Elektrophorese - Einheit bricht und anschließend die wässrige und die organische Phase trennt.

Der Stand der Technik der neutralen Wäsche ist die Dispergierung der organischen Phase mit Waschwasser mittels Rührwerksbehälter oder statischem Mischer. Die anschließende Trennung der Phasen erfolgt in Trennbehältern (Settler mit oder ohne Koaleszer) und unter Umständen unter Zuhilfenahme von Desemulgatoren (Trennhilfen).

Das grundsätzliche Verfahren der Elektrophorese ist in DE-A-2 808 225 beschrieben. Dabei wird eine Emulsion aus Nitrobenzol und Abfallschwefelsäure im Batchverfahren durch Rühren oder Umpumpen dispergiert und anschließend diese Emulsion in einem Elektrodenraum gebrochen.

DE-A-2808225 beschreibt die Aufarbeitung von Nitrobenzol, aus dem überschüssiges Benzol bereits abgetrennt wurde. DE-A-2 808 225 beschreibt jedoch nicht, dass das rohe, mit Benzol behaftete Nitrobenzol vor der elektrophoretischen Brechung der Dispersion einer sauren und dann noch einer alkalischen Wäsche und anschließend noch einer neutralen Wäsche und nach der elektrophoretischen Brechung der Dispersion noch einer Abtrennung von Wasser und Benzol unterzogen werden muss, um eine höhere Reinheit des erzeugten Nitrobenzols zu erhalten. Das Verfahren gemäß DE-A-2 808 225 ist auch nicht ohne weiteres übertragbar auf die Reinigung von rohem Nitrobenzol durch neutrale Wäsche, die im Anschluss an die saure und die alkalische Wäsche erfolgt, weil in diesen Vorstufen zur neutralen Wäsche bereits wesentliche, das Ergebnis der Elektrophorese beeinflussende Bestandteile, wie zum Beispiel die Schwefelsäure, aus dem Rohnitrobenzol ausgewaschen sind.

Die Aufgabe der vorliegenden Erfindung besteht daher darin, ein einfaches und wirtschaftliches Verfahren zur Herstellung von Nitrobenzol zur Verfügung zu stellen, bei dem Nitrobenzol in hoher Reinheit und unter Vermeidung einer Zugabe von Desemulgatoren (Trennhilfen) sowie unter Einsatz geringster Waschwassermengen, erhalten werden kann.

Die Erfindung betrifft ein Verfahren zur Herstellung von Nitrobenzol, bei dem man
a) Benzol mit Nitriersäure zu Nitrobenzol umsetzt, und anschließend
b) das rohe Nitrobenzol in einer sauren Wäsche mit Wasser wäscht, und anschließend
c) das rohe Nitrobenzol in einer alkalischen Wäsche mit Wasser wäscht, und anschließend
d) das rohe Nitrobenzol in einer neutralen Wäsche mit Wasser wäscht, und anschließend
e) aus dem rohen Nitrobenzol Wasser und Benzol entfernt, wobei man gereinigtes Nitrobenzol erhält,
dadurch gekennzeichnet, dass man
in Schritt d) das rohe Nitrobenzol zunächst mit Wasser in einem Mischorgan vermischt und dispergiert, so dass die organische, im wesentlichen Nitrobenzol enthaltende Phase zumindest teilweise dispergiert vorliegt, und man anschließend die Dispersion einer Elektrophorese - Einheit zuführt, in welcher man die Dispersion durch das Anlegen einer elektrischen Gleichspannung bricht, und man anschließend die wässrige und die organische Phase trennt.

Die Nitrierung von Benzol zu Nitrobenzol mit Nitriersäure (Gemisch aus Salpetersäure und Schwefelsäure) in Schritt a) erfolgt dabei üblicherweise nach einem Verfahren aus dem Stand der Technik, z.B. gemäß EP-A-436 443. Dabei kann die Nitrierung unter Abfuhr von Reaktionswärme, z.B. isotherm, oder auch adiabat erfolgen.

Das in Schritt a) hergestellte rohe Nitrobenzol wird üblicherweise zunächst in einem Trennbehälter von überschüssiger Schwefelsäure getrennt. Anschließend wird das rohe Nitrobenzol, das üblicherweise noch Spuren an Schwefelsäure enthält, in einer sauren Wäsche in Schritt b) gewaschen und dann von dem sauren Waschwasser, bevorzugt durch Phasentrennung, getrennt. Bevorzugt hat das eingesetzte saure Waschwasser einen pH-Wert von ≤ 5 (gemessen bei 20°C).

Das rohe Nitrobenzol wird anschließend in einer alkalischen Wäsche in Schritt c) alkalisch gewaschen und anschließend von dem alkalischen Waschwasser, bevorzugt durch Phasentrennung, getrennt. Bevorzugt hat das eingesetzte alkalische Waschwasser einen pH-Wert von ≥ 9 (gemessen bei 20°C). Die anschließende Aufarbeitung des alkalischen Abwassers kann nach den Verfahren nach dem Stand der Technik, z.B. gemäß EP-A-1 593 654 und EP-A-1 132 347 erfolgen.

Das so erhaltene rohe Nitrobenzol weist bevorzugt eine Temperatur von 20°-60°C, besonders bevorzugt von 30°-50°C auf. Bevorzugt enthält das so erhaltene rohe Nitrobenzol 4 bis 10 Gew.-% Benzol, bezogen auf das Gewicht des rohen Nitrobenzols, und weniger als 100 ppm, besonders bevorzugt weniger als 60 ppm, bezogen auf das Gewicht des rohen Nitrobenzols, an Nitrophenolen.

In Schritt d) wird das so erhaltene rohe Nitrobenzol in einer neutralen Wäsche gewaschen. Dabei wird das rohe Nitrobenzol zunächst mit Wasser vermischt und dispergiert, so dass die organische, im Wesentlichen Nitrobenzol enthaltende Phase zumindest teilweise dispergiert vorliegt. Bevorzugt enthält die Dispersion 5 bis 20 Gew.-% Wasser bezogen auf das Gewicht der Dispersion. Dies erfolgt bevorzugt mit einem Rührer oder einem Mischer oder bevorzugt in einer Pumpe, besonders bevorzugt in einer Kreiselpumpe, deren Laufrad sich mit wenigstens 1450 und bevorzugt mit wenigstens 2900 Umdrehungen pro Minute dreht. Der Energieeintrag durch das Mischorgan liegt bevorzugt bei 20 bis 30 kW pro m³. Dabei werden Wassertröpfchen mit einer flächenmittleren Partikelgröße von bevorzugt kleiner 2.500 µm², besonders bevorzugt kleiner 100 µm² und ganz besonders bevorzugt kleiner 10 µm² erzeugt.

Als Waschwasser wird in Schritt d) bevorzugt vollentsalztes Wasser (VE-Wasser), besonders bevorzugt eine Mischung aus VE-Wasser und Kondensat und ganz besonders bevorzugt Dampfkondensat eingesetzt.

Im Anschluss an die Vermischung und Dispergierung wird die Dispersion einer Elektrophorese - Einheit zugeführt, worin die Dispersion ein Gleichspannungsfeld durchläuft. Dabei wird die Dispersion gebrochen, das heißt, die Phasengrenzfläche sinkt und die Phasen separieren sich wieder. In der Elektrophorese - Einheit passiert die Dispersion ein Gleichspannungsfeld von bevorzugt 100 bis 500 Volt, besonders bevorzugt 200 bis 400 Volt und ganz besonders bevorzugt von 220 bis 300 Volt. Die Stromstärke beträgt bevorzugt 0,05 bis 3 Ampere und besonders bevorzugt 0,1 bis 1 Ampere. Durch die kontinuierliche Fahrweise des Waschprozesses wird dafür gesorgt, dass der Elektrodenraum der Elektrophorese - Einheit stets geflutet ist, so dass auch bei eventuellen elektrischen Überschlägen zwischen den Elektroden keine Gefahr einer Zündung von gegebenenfalls zündfähigen Gasgemischen im Elektrodenraum besteht.

Anschließend wird die organische und die wässrige Phase, bevorzugt in einem Trennbehälter, getrennt.

Die Drehzahl der Pumpen für die Erzeugung der Dispersion, die Menge an eingesetztem Waschwasser und die Anzahl der Waschstufen der neutralen Wäsche (umfassend Pumpe, Elektrophorese - Einheit und Trennbehälter) bestimmen dabei den erreichbaren Reinheits-grad des Nitrobenzols in Schritt d).

In Schritt e) wird das rohe Nitrobenzol anschließend von Wasser und Benzol befreit. Dies erfolgt bevorzugt destillativ, wobei über Kopf Wasser und Benzol und ggf. Leichtsieder ausgetrieben werden. Es verbleibt das getrocknete und von Benzol befreite Nitrobenzol, welches noch eine geringe Menge Dinitrobenzol enthalten kann und eine Leitfähigkeit von bevorzugt < 50 µS/cm, besonders bevorzugt < 25 µS/cm und ganz besonders bevorzugt eine Leitfähigkeit von < 10 µS/cm besitzt.

Das erfindungsgemäße Verfahren weist die folgenden Vorteile auf:
Geringer Wasserverbrauch und damit verbunden eine geringe Menge an Abwasser, hohe Reinheit des Endproduktes Nitrobenzol, geringerer Investaufwand durch Einsparung von einer bis zwei Waschstufen je nach gewünschtem Reinigungsgrad, Verzicht auf den Einsatz chemischer Hilfsmittel wie Desemulgatoren, Verfügbarkeit und Zuverlässigkeit des Verfahrens.

### Beispiele

### Beispiel 1 (Vergleichsbeispiel)

Das rohe Nitrobenzol eines isothermen Nitrobenzolprozesses wird zunächst einer sauren Wäsche unterzogen und anschließend unter Zugabe von Natronlauge (50 %ig) in einem Rührwerksbehälter alkalisch gestellt. Danach wird das Gemisch in einem nachgeschalteten Trennbehälter aufgrund der Dichteunterschiede in eine organische Phase (Rohnitrobenzol) und eine wässrige Phase (Ablauge) getrennt. Die wässrige Phase wird in eine separate alkalische Aufarbeitung gefahren. Das verbleibende 31°C warme Rohnitrobenzol, das 95 ppm Dinitrobenzole, 42 ppm Nitrophenole und 100 ppm Monopolbrillantöl enthält und einen Benzolgehalt von 4,8 Gewichtsprozent hat, wird in einer vierstufigen Gegenstromwäsche mit einer Mischung aus VE-Wasser und Dampfkondensat neutralgewaschen. Dazu werden 4 Rührwerksbehälter mit nachgeschalteten Trennapparaten eingesetzt (Mixer- / Settler- Technologie). Das so gewaschene rohe Nitrobenzol wird abschließend aus dem letzten Trennbehälter zum Trocknen und zum Abtrennen von Benzol und Leichtsiedern einer Destillation zugeführt. Das Endprodukt enthält 30 ppm Restfeuchte, 99 ppm Dinitrobenzole, 2 ppm Nitrophenole, < 10 ppm Benzol und eine Leitfähigkeit von 20 µS/cm.

### Beispiel 2 (Vergleichsbeispiel)

Das Rohnitrobenzol eines adiabatischen Nitrobenzolprozesses wird zunächst einer sauren Wäsche zugeführt und anschließend unter Zugabe von Natronlauge (50 %ig) in einem Rührwerksbehälter alkalisch gestellt. Danach wird das Gemisch in einem nachgeschalteten Trennbehälter aufgrund der Dichteunterschiede in eine organische Phase (Rohnitrobenzol) und eine wässrige Phase (Ablauge) getrennt. Die wässrige Phase wird in eine separate alkalische Aufarbeitung gefahren. Das verbleibende 40°C warme Rohnitrobenzol, das 245 ppm Dinitrobenzole und 53 ppm Nitrophenole enthält und einen Benzolgehalt von 7,5 Gewichtsprozent hat, wird in einer vierstufigen Gegenstromwäsche neutralgewaschen. Dazu werden 4 Rührwerksbehälter (wobei nur die ersten 3 Behälter gerührt sind) mit nachgeschalteten Trennapparaten eingesetzt (Mixer- / Settler-Technologie). Zusätzlich wird ab der zweiten Waschstufe Monopolbrillantöl (Einsatzmenge sind 10 ppm Monopolbrillantöl bezogen auf Nitrobenzol) als Trennhilfe eingesetzt. Das so gewaschene rohe Nitrobenzol wird abschließend aus dem letzten Trennbehälter zum Trocknen und zum Abtrennen von Benzol und Leichtsiedern einer Destillation zugeführt. Das Endprodukt enthält 30 ppm Restfeuchte, 252 ppm Dinitrobenzole, 3 ppm Nitrophenole, < 10 ppm Benzol und eine Leitfähigkeit von 10 µS/cm.

### Beispiel 3 (erfindungsgemäßes Beispiel)

Das Rohnitrobenzol eines adiabatischen Nitrobenzolprozesses wird zunächst einer sauren Wäsche zugeführt und anschließend unter Zugabe von Natronlauge (32 %ig) in einem Statikmischer alkalisch gestellt. Danach wird das Gemisch in einem nachgeschalteten Trennbehälter aufgrund der Dichteunterschiede in eine organische Phase (Rohnitrobenzol) und eine wässrige Phase (Ablauge) getrennt. Das 39°C warme Rohnitrobenzol, das 179 ppm Dinitrobenzole und 51 ppm Nitrophenole enthält und einen Benzolgehalt von 6,8 Gewichtsprozent hat, wird in einer ersten neutralen Wäsche mit Waschwasser aus der zweiten neutralen Wäsche auf der Saugseite einer Kreiselpumpe, die mit 2900 Umdrehungen pro Minute betrieben wird, vereinigt. In dieser Kreiselpumpe findet die erste neutrale Wäsche des rohen Nitrobenzols statt. Die dabei entstehende Dispersion wird über eine Elektrophorese - Einheit, die mit einer Gleichspannung von 280 V bei 0,3 A betrieben wird, gebrochen und in einen nachgeschalteten Trennbehälter gefahren. Anschließend wird das rohe Nitrobenzol aus diesem Trennbehälter in der zweiten neutralen Wäsche mit Waschwasser aus der dritten neutralen Wäsche auf der Saugseite einer Kreiselpumpe, die mit 2900 Umdrehungen pro Minute betrieben wird, vereinigt. In dieser Kreiselpumpe findet die zweite neutrale Wäsche des rohen Nitrobenzols statt. Die dabei entstehende Dispersion wird über eine zweite Elektrophorese - Einheit, die mit einer Gleichspannung von 240 V bei 0,4 A betrieben wird, gebrochen und in einen nachgeschalteten Trennbehälter gefahren. Anschließend wird das rohe Nitrobenzol aus diesem Trennbehälter in der dritten neutralen Wäsche mit Waschwasser, bestehend aus einer Mischung aus VE-Wasser und Dampfkondensat, auf der Saugseite einer Kreiselpumpe, die mit 2900 Umdrehungen pro Minute betrieben wird, vereinigt. In dieser Kreiselpumpe findet die dritte neutrale Wäsche des rohen Nitrobenzols statt. Die dabei entstehende Dispersion wird über eine dritte Elektrophorese - Einheit, die mit einer Gleichspannung von 290 V und 0,9 A betrieben wird, gebrochen und in einen nachgeschalteten Trennbehälter gefahren. Das so gewaschene rohe Nitrobenzol wird abschließend aus dem letzten Trennbehälter zum Trocknen und zum Abtrennen von Benzol und Leichtsiedern einer Destillation zugeführt. Das so erhaltene Nitrobenzol enthält 41 ppm Restfeuchte, 192 ppm Dinitrobenzole, 3 ppm Nitrophenole, < 2 ppm Benzol und eine Leitfähigkeit von 6 µS/cm.

### Beispiel 4 (erfindungsgemäßes Beispiel)

Das Rohnitrobenzol eines adiabatischen Nitrobenzolprozesses wird zunächst einer sauren Wäsche zugeführt und anschließend unter Zugabe von Natronlauge (32%ig) in einem Statikmischer alkalisch gestellt. Danach wird das Gemisch in einem nachgeschalteten Trennbehälter aufgrund der Dichteunterschiede in eine organische Phase (Rohnitrobenzol) und eine wässrige Phase (Ablauge) getrennt. Das 41°C warme Rohnitrobenzol, das 184 ppm Dinitrobenzole und 55 ppm Nitrophenole enthält und einen Benzolgehalt von 6,3 Gewichtsprozent hat, wird in der ersten neutralen Wäsche mit Waschwasser aus der zweiten neutralen Wäsche auf der Saugseite einer Kreiselpumpe, die mit 2900 Umdrehungen pro Minute betrieben wird, vereinigt. In dieser Kreiselpumpe findet die erste neutrale Wäsche des rohen Nitrobenzols statt. Die dabei entstehende Dispersion wird über eine Elektrophorese - Einheit, die mit einer Gleichspannung von 280 V bei 0,3 A betrieben wird, gebrochen und in einen nachgeschalteten Trennbehälter gefahren. Anschließend wird das rohe Nitrobenzol aus diesem Trennbehälter in der zweiten neutralen Wäsche mit Waschwasser, bestehend aus einer Mischung aus VE-Wasser und Dampfkondensat, auf der Saugseite einer Kreiselpumpe, die mit 2900 Umdrehungen pro Minute betrieben wird, vereinigt. In dieser Kreiselpumpe findet die zweite neutrale Wäsche des rohen Nitrobenzols statt. Die dabei entstehende Dispersion wird über eine zweite Elektrophorese - Einheit, die mit einer Gleichspannung von 280 V und 0,6 A betrieben wird, gebrochen und in einen nachgeschalteten Trennbehälter gefahren. Das so gewaschene rohe Nitrobenzol wird abschließend aus dem letzten Trennbehälter zum Trocknen und zum Abtrennen von Benzol und Leichtsiedern einer Destillation zugeführt. Das Endprodukt enthält 45 ppm Restfeuchte, 196 ppm Dinitrobenzole, 6 ppm Nitrophenole, < 2 ppm Benzol und eine Leitfähigkeit von 23 µS/cm.

Die wesentlichen Daten und Ergebnisse der Beispiele sind in der nachfolgenden Tabelle nochmals zusammengefasst. Darin ist auch angegeben, wie viel Waschwasser pro Tonne Nitrobenzol (NB) in der neutralen Wäsche in Schritt d) eingesetzt wird.

Die Leitfähigkeit des rohen Nitrobenzols vor der neutralen Wäsche beträgt in allen Beispielen > 1000 µS/cm.

| Beispiel | Verfahren | Waschstufen Anzahl | Waschwasser (m³/t NB) | Elektrophoresen .Anzahl | Brilliantöl Ja / Nein | Leitfähigkeit Endprodukt |
|---|---|---|---|---|---|---|
| 1 | Rührer / Settler | 4 | 0,23 | 0 | ja | 20 µS/cm |
| 2 | Rührer / Settler | 4 | 0,3 | 0 | ja | 10 µS/cm |
| 3 | Pumpe / Settler | 3 | 0,16 | 3 | nein | 6 µS/cm |
| 4 | Pumpe / Settler | 2 | 0,16 | 2 | nein | 23 µS/cm |

Das verwendete Trennmittel (Beispiele 1 und 2) ist ein Natriumsalz eines sulfatierten Rizinusöls mit dem Handelsnamen Monopolbrillantöl der Fa. Degussa, Standort Krefeld, Stockhausen GmbH.

Leitfähigkeitsbestimmungsmethode: Messung von ionischen Spurenkomponenten in Nitrobenzol durch Bestimmung der elektrische Leitfähigkeit des wässrigen Extraktes (in Anlehnung an DIN 53779 "Bestimmung der elektrischen Leitfähigkeit und des spezifischen elektrischen Widerstandes von wässrigen Auszügen"). Die Reinheit von Nitrobenzol wird bestimmt, indem die im Endprodukt enthaltenen ionischen Spurenkomponenten, wie z.B. Natriumsulfat, Natriumnitrat oder auch Nitrophenole mit Wasser extrahiert werden, um dann die elektrische Leitfähigkeit des wässrigen Extraktes per Konduktometer zu messen. Die Reinheit von Nitrobenzol wird in µS/cm angegeben. Die Leitfähigkeit kann mit einem Messgerät, wie z.B. dem Konduktometer der Firma Knick Typ 702, gemessen werden. Ausführung: 500 ml Nitrobenzolprobe werden mit 25 ml demineralisiertem Wasser extrahiert. Anschließend wird die wässrige Phase von der organischen Phase abgetrennt. Abschließend erfolgt die Bestimmung der Leitfähigkeit der wässrigen Phase mit einem Konduktometer.

Dabei zeigt eine niedrige Leitfähigkeit eine hohe Reinheit der Nitrobenzol-Probe an.

## Patentansprüche

1. Verfahren zur Herstellung von Nitrobenzol, bei dem man
a) Benzol mit Nitriersäure zu Nitrobenzol umsetzt, und anschließend
b) das rohe Nitrobenzol in einer sauren Wäsche mit Wasser wäscht, und anschließend
c) das rohe Nitrobenzol in einer alkalischen Wäsche mit Wasser wäscht, und anschließend
d) das rohe Nitrobenzol in einer neutralen Wäsche mit Wasser wäscht, und anschließend
e) aus dem rohen Nitrobenzol Wasser und Benzol entfernt, wobei man gereinigtes Nitrobenzol erhält,
**dadurch gekennzeichnet, dass man**
in Schritt d) das rohe Nitrobenzol zunächst mit Wasser in einem Mischorgan vermischt und dispergiert, so dass die organische, im wesentlichen Nitrobenzol enthaltende Phase zumindest teilweise dispergiert vorliegt, und man anschließend die Dispersion einer Elektrophorese - Einheit zuführt, in welcher man die Dispersion durch das Anlegen einer elektrischen Gleichspannung bricht, und man anschließend die wässrige und die organische Phase trennt.

2. Verfahren nach Anspruch 1, bei dem die neutrale Wäsche in Schritt d) mehrstufig ausgeführt ist, wobei jede Stufe über ein Mischorgan zur Vermischung des rohen Nitrobenzols mit Wasser und eine nachgeschaltete Elektrophorese - Einheit verfügt, und wobei das Waschwasser aus der nachfolgenden Waschstufe als Wasser für die vorangegangene Waschstufe dient und wobei in die letzte Stufe frisches Wasser aufgegeben wird.

## Claims

1. A process for the production of nitrobenzene, in which
a) benzene is reacted with nitrating acid to form nitrobenzene, and then
b) the crude nitrobenzene is washed with water in an acidic wash, and then
c) the crude nitrobenzene is washed with water in an alkaline wash, and then
d) the crude nitrobenzene is washed with water in a neutral wash, and then
e) water and benzene are removed from the crude nitrobenzene to obtain purified nitrobenzene,
**characterised in that**
in step d) the crude nitrobenzene is first mixed with water in a mixer and dispersed so that the organic phase substantially containing nitrobenzene is present in at least partly dispersed form, and the dispersion is then fed into an electrophoresis unit in which the dispersion is broken by applying a direct-current electric voltage and the aqueous and organic phases are then separated.

2. The process according to claim 1, in which the neutral wash in step d) is carried out in multiple steps, wherein each step has a mixer for mixing the crude nitrobenzene with water and a downstream electrophoresis unit, and wherein the washing water from the subsequent washing step is used as water for the preceding washing step, and wherein fresh water is fed into the final step.

## Revendications

1. Procédé de préparation de nitrobenzène, dans lequel
a) on transforme du benzène avec de l'acide nitrique en nitrobenzène, et ensuite
b) on lave le nitrobenzène brut dans un milieu acide avec de l'eau, et ensuite
c) on lave le nitrobenzène brut dans un milieu alcalin avec de l'eau, et ensuite
d) on lave le nitrobenzène brut dans un milieu neutre avec de l'eau, et ensuite
e) on élimine du nitrobenzène brut, l'eau et le benzène pour obtenir du nitrobenzène purifié,
**caractérisé en ce que**
l'on mélange et disperse dans l'étape d), le nitrobenzène brut, d'abord avec de l'eau dans un appareil de mélange, de sorte que la phase organique contenant essentiellement le nitrobenzène est présente au moins partiellement dispersée et l'on conduit ensuite la dispersion vers une unité d'électrophorèse, dans laquelle on rompt la dispersion par l'imposition d'une tension électrique continue, et l'on sépare ensuite, les phases aqueuse et organique.

2. Procédé selon la revendication 1, dans lequel le lavage neutre est réalisé à l'étape d) en plusieurs étapes, où chaque étape dispose d'un appareil de mélange pour le mélange du nitrobenzène brut avec de l'eau et une unité suivante d'électrophorèse, où l'eau de lavage de l'étape de lavage suivante sert d'eau pour l'étape de lavage précédente et où de l'eau fraîche est introduite dans la dernière étape
